# NEW EUROPEAN PATENT SPECIFICATION

(11) **EP 1 814 527 B2**
(45) Date of publication and mention of the opposition decision: **18.11.2020**
(45) Mention of the grant of the patent: 18.12.2013
(21) Application number: 05810652.7
(22) Date of filing: 29.10.2005
(51) Int. Cl.: A61K 9/20, A61K 31/4184, A61K 31/4422, A61P 9/00, A61P 9/04, A61P 9/08, A61P 9/10, A61P 9/12, A61P 3/10, A61P 25/28

(54) **BILAYER TABLET COMPRISING TELMISARTAN AND AMLODIPINE**
DOPPELSCHICHTTABLETTE MIT TELMISARTAN UND AMLODIPIN
COMPRIME BICOUCHE CONTENANT DU TELMISARTAN ET DE L'AMLODIPINE

(30) Priority: 05.11.2004 EP 04026234
(43) Date of publication of application: 08.08.2007
(60) Divisional application: 14170165.6
(73) Proprietor: Boehringer Ingelheim International GmbH, 55216 Ingelheim (DE); Boehringer Ingelheim Pharma GmbH & Co. KG, 55216 Ingelheim (DE)
(72) Inventor: EISENREICH, Wolfram, 89081 Ulm (DE)
(86) International application number: PCT/EP2005/011596
(87) International publication number: WO 2006/048208

(56) References cited:
- WO-A-03/059327
- WO-A-03/097045
- WO-A-2005/070462
- WO-A1-00/27397
- WO-A1-03/059327
- US-A- 6 071 939
- US-A- 6 162 802
- STANGIER, J. ET AL: "PHARMACOKINETICS OF REPEATED ORAL DOSES OF AMLODIPINE AND AMLODIPINE PLUS TELMISARTAN IN HEALTHY VOLUNTEERS" JOURNAL OF CLINICAL PHARMACOLOGY, LIPPINCOTT CO, HAGERSTOWN, MD, US, vol. 40, no. 12, PART 1, December 2000 (2000-12), pages 1347-1354, XP008058908 ISSN: 0091-2700
- NEUTEL, J. M. ET AL: "The efficacy and safety of telmisartan compared to enalapril in patients with severe hypertension" INTERNATIONAL JOURNAL OF CLINICAL PRACTICE, vol. 53, no. 3, 1999, pages 175-178, XP008060086
- Abdoh, A. et al: Pharm. Dev. Technol., vol. 9, no. 1, 2004, pages 15-24,

## Description

The present invention relates to a pharmaceutical tablet as in claim 1 comprising a first layer of the angiotensin II receptor antagonist telmisartan in a dissolving tablet matrix and a second layer of the calcium channel blocker amlodipine in a disintegrating or eroding tablet matrix.

### Background of the invention

Telmisartan is an angiotensin II receptor antagonist developed for the treatment of hypertension and other medical indications as disclosed in EP-A-502314. Its chemical name is 4'-[2-n-propyl-4-methyl-6-(1-methylbenzimidazol-2-yl)-benzimidazol-1-ylmethyl]-biphenyl-2-carboxylic acid having the following structure:

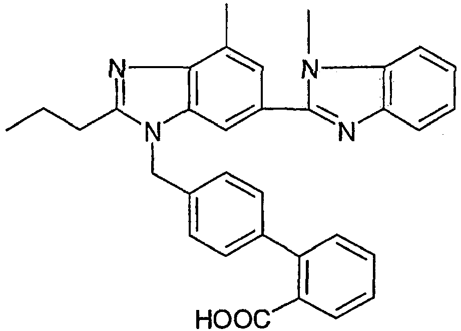

Telmisartan is manufactured and supplied in the free acid form. It is characterized by its very poor solubility in aqueous systems at the physiological pH range of the gastro-intestinal tract between pH 1 to 7. As disclosed in WO 00/43370, crystalline telmisartan exists in two polymorphic forms having different melting points. Under the influence of heat and humidity, the lower melting polymorph B transforms irreversibly into the higher melting polymorph A.

Amlodipine was first disclosed in EP-A-89167. It belongs to the group of calcium channel blockers and its chemical name is 3-ethyl-5-methyl-2-(2-aminoethoxymethyl)-4-(2-chlorophenyl)-1,4-dihydro-6-methyl-3,5-pyridinedicarboxylate, C₂₀H₂₅ClN₂O₅, M_{R} 408,88,having the following stucture:

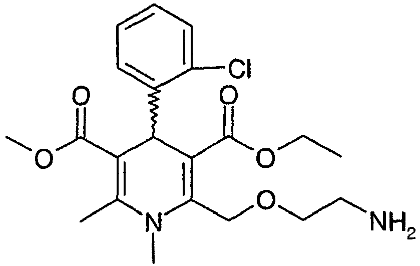

Pharmaceutically amlodipine is used as maleate (C₂₄H₂₉ClN₂O₉; M_{R} 524,96), benzenesulfonate or besylate (C₂₆H₃₁ClN₂O₈S; M_{R} 567,10; EP-A-244944) and mesylate salt (C₂₁H₂₆ClN₂O₈S; M_{R} 502,01).

"Calcium channel blockers" are also called "calcium antagonists" or "calcium blockers". They are medications that decrease the heart's pumping strength and relax blood vessels. They are used to treat high blood pressure, angina (chest pain or discomfort caused by reduced blood supply to the heart muscle) and some arrhythmias.

Stangier, J. et al, "Pharmacokinetics of repeated oral doses of amlodipine and amlodipine plus telmisartan in healthy volunteers", Journal of Clinical Pharmacology, vol. 40 (12), part 1, December 2000, pages 1347-1354, discloses the concomitant administration of tablets of telmisartan and tablets of amlodipine.

### Object of the invention

The mechanisms of action of telmisartan and amlodipine are considered to cooperate favourably in the treatment of hypertension particularly in patients where the target blood pressure cannot be achieved with one of the medications only. There is an increasing desire for a fixed dose combination product comprising the active ingredients telmisartan and amlodipine. However, both telmisartan and amlodipine are chemical compounds difficult to handle. Therefore, an oral fixed dose combination dosage form which combines the features of pharmacologic efficacy, adequate drug stability and a reliable and robust method of manufacture has to overcome a number of technical problems. It is an object of the present invention to provide such a fixed dose combination dosage form.

There are various types of fixed dose combination dosage forms conceivable but it cannot be predicted which of these dosage forms combines product stability, pharmacological efficacy and reliable manufacture best. Generally, combinations of two active pharmaceutical ingredients could be formulated as oral solid or oral liquid dosage forms such as tablets, capsules, coated or sugar-coated tablets, granules, oral solutions, emulsions or suspensions, syrups and lozenges. In view of experiences with liquid dosage forms of telmisartan oral liquid dosage forms are not considered a preferred embodiment according to the present invention. An instant release oral solid dosage form containing two drugs could be prepared by either making a powder mixture or a co-granulate of the two active ingredients with the necessary excipients. However, for a combination of telmisartan and amlodipine, this approach turns out not to result in a dosage form with sufficient product stability. A telmisartan formulation with acceptable in vivo performance has to comprise basic components like for example sodium hydroxide or meglumine whereas amlodipine is surprisingly not stable enough when it gets in direct contact with excipients to be used in a telmisartan formulation. The ester bonds in the amlodipine molecule appear to be subject to hydrolysis when exposed to an alkaline milieu. Therefore, the standard approach of directly mixing the active components with the necessary excipients cannot be applied to a fixed dose combination of telmisartan and amlodipine and more sophisticated techniques are needed to separate the basic telmisartan formulation from the amlodipine drug substance. Under these circumstances perlonget, coating or bilayer tablet technology could be used.

The perlonget approach is to produce separate film-coated tablets for telmisartan and amlodipine in such a size and shape that these can be filled into capsules. It turns out that large capsule sizes like 0 or bigger would be required for the high dose combinations, which is not preferable with regard to patients' compliance.

Another approach is to apply a film coat to the pure amlodipine drug substance or to granules/pellets containing amlodipine. Surprisingly the so coated particles are not stable in the alkaline and hygroscopic milieu of the telmisartan formulation.

The present invention is based on the recognition, that the dosage form, which combines adequate drug stability, optimum drug release of both active ingredients, pharmacological efficacy and reliable manufacture for a combination of telmisartan and amlodipine best, is a bilayer tablet.

### Summary of the invention

In accordance with the present invention problems associated with the preparation of a fixed dose combination drug comprising telmisartan and amlodipine can best be handled by means of a bilayer pharmaceutical tablet as in claim 1 comprising a first layer of telmisartan, preferably in substantially amorphous form, in a dissolving tablet matrix and a second layer of amlodipine in a disintegrating or eroding tablet matrix.

The tablet according to the present invention provides a largely pH-independent dissolution of the poorly water-soluble telmisartan, thereby facilitating dissolution of the drug at a physiological pH level, and adequate stability and drug release of amlodipine. The tablet structure also overcomes the stability problem caused by the incompatibility of amlodipine with basic constituents of the telmisartan formulation.

### Definitions

As used herein, the term "substantially amorphous" refers to a product comprising amorphous constituents in a proportion of at least 90%, preferably at least 95%, as determined by X-ray powder diffraction measurement.

The term "dissolving tablet matrix" refers to a pharmaceutical tablet base formulation having instant release (fast dissolution) characteristics that readily dissolves in a physiological aqueous medium.

The term "disintegrating or eroding tablet matrix" refers to a pharmaceutical tablet base formulation having instant release characteristics that readily disintegrates or erodes in a physiological aqueous medium.

### Description of the invention

A fixed dose combination according to the present invention represents a pharmaceutical bilayer tablet comprising a first layer of telmisartan in substantially amorphous form and a second layer of amlodipine in a disintegrating or eroding tablet matrix.

The active ingredient telmisartan is generally supplied in its free acid form, although pharmaceutically acceptable salts such as the sodium salt may also be used. Since during subsequent processing telmisartan is normally dissolved and transformed into a substantially amorphous form, its initial crystal morphology and particle size are of little importance for the physical and biopharmaceutical properties of the bilayer tablet formulation obtained. It is, however, preferred to remove agglomerates from the starting material, e.g. by sieving, in order to facilitate wetting and dissolution during further processing.

Substantially amorphous telmisartan may be produced by any suitable method known to those skilled in the art, for instance, by freeze drying of aqueous solutions, coating of carrier particles in a fluidized bed, and solvent deposition on sugar pellets or other carriers. Preferably, however, the substantially amorphous telmisartan is prepared by the specific spray-drying method described in WO 03/059327.

A bilayer tablet according to the present invention generally contains 10 to 160 mg, preferably 20 to 80 mg or 40 to 80 mg, of telmisartan; and 1 to 20 mg, preferably 2.5 to 10 mg, of amlodipine.

Preferred dose strengths of telmisartan are 20 mg, 40 mg and 80 mg; preferred dose strengths of amlodipine are 2.5 mg, 5 mg and 10 mg.

Presently preferred forms are bilayer tablets comprising 20/10 mg, 40/10 mg, 80/10 mg, 20/5 mg, 40/5 mg, 80/5 mg, 20/2.5 mg, 40/2.5 mg and 80/2.5 mg of telmisartan and amlodipine, respectively.

The first tablet layer contains telmisartan in substantially amorphous form dispersed in a dissolving tablet matrix having instant release (fast dissolution) characteristics.

The dissolving tablet matrix of the telmisartan layer comprises a basic agent, a water-soluble diluent and, optionally, other excipients and adjuvants.

Specific examples of suitable basic agents are alkali metal hydroxides such as NaOH and KOH; basic amino acids such as arginine and lysine; and meglumine (N-methyl-D-glucamine), NaOH and meglumine being preferred.

Specific examples of suitable water-soluble diluents are carbohydrates such as monosaccharides like glucose; oligosaccharides like sucrose, anhydrous lactose and lactose monohydrate; and sugar alcohols like sorbitol, mannitol, erythrol and xylitol. Sorbitol is a preferred diluent.

The other excipients and/or adjuvants are, for instance, selected from binders, carriers, fillers, lubricants, flow control agents, crystallization retarders, solubilizers, coloring agents, pH control agents, surfactants and emulsifiers, specific examples of which are given below in connection with the second tablet layer composition. The excipients and/or adjuvants for the first tablet layer composition are preferably chosen such that a non-acidic, fast dissolving tablet matrix is obtained.

The first tablet layer composition generally comprises 3 to 50 wt.%, preferably 5 to 35 wt.%, of active ingredient; 0.25 to 20 wt.%, preferably 0.40 to 15 wt.%, of basic agent; and 30 to 95 wt.%, preferably 60 to 80 wt.% of water-soluble diluent (filler). Other (optional) constituents may, for instance, be chosen from one or more of the following excipients and/or adjuvants in the amounts indicated:
10 to 30 wt.%, preferably 15 to 25 wt.%, of binders, carriers and fillers, thereby replacing the water-soluble diluent;
0.1 to 5 wt.%, preferably 0.5 to 3 wt.%, of lubricants;
0.1 to 5 wt.%, preferably 0.3 to 2 wt.%, of flow control agents;
1 to 10 wt.%, preferably 2 to 8 wt.%, of crystallization retarders;
1 to 10 wt.%, preferably 2 to 8 wt.%, of solubilizers;
0.05 to 1.5 wt.%, preferably 0.1 to 0.8 wt.%, of coloring agents;
0.5 to 10 wt.%, preferably 2 to 8 wt.%, of pH control agents;
0.01 to 5 wt.%, preferably 0.05 to 1 wt.%, of surfactants and emulsifiers.

The second tablet layer composition comprises amlodipine dispersed in a disintegrating or eroding tablet matrix having instant release (fast dissolution) characteristics. The disintegrating or eroding tablet matrix may have weakly acidic, neutral or weakly basic properties, a neutral tablet matrix being preferred.

In a preferred embodiment, the disintegrating or eroding matrix comprises one or more fillers, a disintegrant, a lubricant and, optionally flow control agents, binders or polymers, other excipients and adjuvants.

Preferred fillers for the second layer are selected from the group consisting of pregelatinized starch, microcrystalline cellulose, cellulose, mannitol, erythritol, lactose monohydrate, dibasic calcium phosphate anhydrous, sorbitol, and xylitol. Particularly preferred are pregelatinized starch, microcrystalline cellulose, dibasic calcium phosphate anhydrous and lactose monohydrate.

Preferred lubricants are sodium stearyl fumarate and magnesium stearate. Particularly preferred is magnesium stearate.

Preferred disintegrants are selected from the group consisting of croscarmellose sodium (crosslinked carboxymethylcellulose sodium), sodium starch glycolate, crospovidone (crosslinked polyvinylpyrrolidone), corn starch, pregelatinized starch, low-substituted hydroxypropylcellulose and microcrystalline cellulose. Particularly preferred are sodium starch glycolate and crospovidone.

Preferred binders are selected from the group consisting of polyvinylpyrrolidone (Povidone), copolymers of vinylpyrrolidone with other vinylderivatives (Copovidone), microcrystalline cellulose, hydroxypropylmethylcellulose, methylcellulose, hydroxypropylcellulose and pregelatinized starch. Particularly preferred are hydroxypropylmethylcellulose and povidone.

Particularly preferred fillers of the second tablet layer composition are pregelatinized starch and/or microcrystalline cellulose as these fillers can additionally serve the purpose of a binder or disintegrant.

Preferred flow control agents are colloidal silicon dioxide and talc. Particularly preferred is colloidal silicon dioxide.

The other excipients and adjuvants, if used, are for example coloring agents including dyes and pigments such as iron oxides.

The second tablet layer composition generally comprises 0.5 to 20 wt.%, preferably 1 to 10 wt.% of amlodipine and 50 to 99.5 wt.%, preferably 80 to 99 wt.% of fillers. The other excipients and/or adjuvants are, for instance, selected from binders (0 to 7 wt. %, preferably 1 to 5 wt. %), disintegrants (0 to 10 wt. %, preferably 1 to 5 wt. %), lubricants (0.25 to 3 wt. %, preferably 0.5 to 2 wt. %), flow control agents (0.25 to 3 wt. %, preferably 0.5 to 2 wt. %) and coloring agents (0.05 to 3 wt. %, preferably 0.1 to 1 wt. %), specific examples of which are also given below. The excipients and/or adjuvants for the second tablet layer composition are preferably chosen such that a neutral, disintegrating or eroding tablet matrix is obtained.

As solvent for the granulation liquid, which, as a volatile component, does not remain in the final product, methanol, ethanol, isopropyl alcohol or purified water can be used; preferred solvents are ethanol and purified water.

The layers can be differentiated by using different colors.

For preparing a bilayer tablet according to the present invention, the first and second tablet layer compositions may be compressed in the usual manner in a bilayer tablet press, e.g. a high-speed rotary press in a bilayer tabletting mode. However, care should be taken not to employ an excessive compression force for the first tablet layer. Preferably, the ratio of the compression force applied during compression of the first tablet layer to the compression force applied during compression of both the first and second tablet layers is in the range of from 1:10 to 1:2. For instance, the first tablet layer may be compressed at moderate force of 4 to 8 kN, whereas the main compression of first plus second layer is performed at a force of 10 to 20 kN. During bilayer tablet compression adequate bond formation between the two layers is achieved by virtue of distance attraction forces (intermolecular forces) and mechanical interlocking between the particles.

The bilayer tablets obtained release the active ingredients rapidly and in a largely pH-independent fashion, with complete release occurring within less than 60 min and release of the major fraction occurring within less than 15 min.

In accordance with the present invention, a substantially increased dissolution rate of the active ingredients and, in particular, of telmisartan is achieved. Normally, at least 70% and typically at least 90% of the drug load are dissolved after 30 min.

The bilayer tablets of the present invention tend to be slightly hygroscopic and are therefore preferably packaged using a moisture-proof packaging material such as aluminium foil blister packs, or polypropylene tubes and HDPE bottles which preferably contain a desiccant.

A preferred method of producing the bilayer tablet according to the present invention comprises
(i) providing a first tablet layer composition by
   a) preparing an aqueous solution of telmisartan, at least one basic agent and, optionally, a solubilizer and/or a crystallization retarder;
   b) spray-drying said aqueous solution to obtain a spray-dried granulate;
   c) mixing said spray-dried granulate with a water-soluble diluent to obtain a premix;
   d) mixing said premix with a lubricant to obtain a final blend for the first layer;
   e) optionally, adding other excipients and/or adjuvants in any of steps a) to d);
(ii) providing a second tablet layer composition by
   a) a direct compression process comprising the steps of
      - blending the active ingredient amlodipine or a pharmaceutically acceptable salt thereof, one or more fillers, flow control agents and disintegrants and/or other excipients in a mixer;
      - optionally dry screening the mixture through a screen in order to segregate cohesive particles and to improve content uniformity;
      - blending the mixture with remaining excipients such as a lubricant in a mixer in order to obtain the final composition;
   b) a wet granulation process comprising the steps of
      - blending the active ingredient amlodipine or a pharmaceutically acceptable salt thereof, one or more fillers, binders, disintegrants and optionally other excipients in a mixer;
      - granulating the mixture by adding granulation liquid, preferably water;
      - wet screening of the granules through a screen in order to segregate bigger agglomerates;
      - drying of the granules in a fluidized bed dryer or a vacuum tray dryer;
      - optionally dry screening of the granules through a screen in order to segregate cohesive particles and to improve content uniformity;
      - blending the granules with remaining excipients such as lubricant(s) in a mixer in order to obtain the final composition.
   c) a dry granulation process comprising the steps of
      - blending the active ingredient amlodipine or a pharmaceutically acceptable salt thereof with either a portion of the filler(s), disintegrant(s), binder(s), flow control agent(s) and lubricant(s) or all the excipients in a mixer;
      - compaction of the mixture on a suitable roller compactor;
      - reducing the ribbons obtained in the previous step to small granules by suitable milling or sieving steps;
      - optionally blending the granules with remaining excipients in a mixer in order to obtain the final composition.
(iii) compressing the first and second tablet layer composition from step (i) and (ii) on a suitable tablet press to form a bilayer tablet.

To provide a first tablet layer composition an aqueous alkaline solution of telmisartan is prepared by dissolving the active ingredient in purified water with the help of one or more basic agents like sodium hydroxide and meglumine. Optionally, a solubilizer and/or a recrystallization retarder may be added. The dry matter content of the starting aqueous solution is generally 10 to 40 wt.%, preferably 20 to 30 wt.%.

The aqueous solution is then spray-dried at room temperature or preferably at increased temperatures of, for instance, between 50 and 100°C in a co-current or countercurrent spray-drier at a spray pressure of, for instance, 1 to 4 bar. Generally speaking, the spray-drying conditions are preferably chosen in such a manner that a spray-dried granulate having a residual humidity of ≤ 5 wt.%, preferably ≤ 3.5 wt.%, is obtained in the separation cyclone. To that end, the outlet air temperature of the spray-drier is preferably kept at a value between about 80 and 90°C while the other process parameters such as spray pressure, spraying rate, inlet air temperature, etc. are adjusted accordingly.

The spray-dried granulate obtained is preferably a fine powder having the following particle size distribution:
- d₁₀ :: ≤ 20 µm, preferably ≤ 10 µm
- d₅₀ :: ≤ 80 µm, preferably 20 to 55 µm
- d₉₀ :: ≤ 350 µm, preferably 50 to 150 µm

After spray-drying, the active ingredient telmisartan as well as the excipients contained in the spray-dried granulate are in a substantially amorphous state with no crystallinity being detectable. From a physical point of view, the spray-dried granulate is a solidified solution or glass having a glass transition temperature Tg of preferably > 50°C, more preferably > 80°C.

Based on 100 parts by weight of active ingredient telmisartan, the spray-dried granulate preferably contains 5 to 200 parts by weight of basic agent and, optionally, solubilizer and/or crystallization retarder.

The water-soluble diluent is generally employed in an amount of 30 to 95 wt.%, preferably 60 to 80 wt.%, based on the weight of the first tablet layer composition. The lubricant is generally added to the premix in an amount of 0.1 to 5 wt.%, preferably 0.3 to 2 wt.%, based on the weight of the first tablet layer composition. Mixing is carried out in two stages, i.e. in a first mixing step the spray-dried granulate and the diluent are admixed using , e.g., a high shear mixer or a free fall blender, and in a second mixing step the lubricant is blended with the premix, preferably also under conditions of high shear. The method of the invention is however not limited to these mixing procedures and, generally, alternative mixing procedures may be employed in steps c), d), and also in the subsequent steps f) and g), such as, e.g., container mixing with intermediate screening.

To provide a second tablet layer composition comprising amlodipine, several different manufacturing methods can be used, for example the direct compression, wet granulation or roller compaction process.

The present invention is preferably directed to a method of manufacturing the second tablet layer composition of amlodipine by a direct compression process comprising the steps of
(1) producing a composition consisting of the active ingredient amlodipine or a pharmaceutically acceptable salt thereof, one or more fillers like e.g. microcrystalline cellulose, dibasic calcium phosphate anhydrous or pregelatinized starch, a disintegrant like sodium starch glycolate or crospovidone, a flow control agent like colloidal silicon dioxide and/or other excipients by mixing the components in a mixer;
(2) optionally dry screening the composition of step (1) through a screen in order to segregate cohesive particles and to improve content uniformity; and
(3) mixing the composition of step (2) and remaining excipients such as the lubricant magnesium stearate in a mixer.

In case of wet granulation amlodipine or a pharmaceutically acceptable salt thereof is premixed in a high shear granulator with suitable fillers such as microcrystalline cellulose, lactose monohydrate or dibasic calcium phosphate anhydrous, and wet binding agents such as hydroxypropylmethylcellulose or povidone, disintegrants such as crospovidone and optionally other suitable excipients. Agglomeration of the powder is promoted through the addition of the granulation liquid (for example purified water or ethanol). After high shear granulation the granulate is wet screened through an appropriate sieve and subsequently dried using a fluid bed dryer or a vacuum tray dryer. The dried granules are optionally dry screened through an appropriate sieve. After addition of the lubricant (for example magnesium stearate) and/ or other excipients, the mixture is blended in a free fall blender or a high shear mixer.

Alternative methods for wet granulation of active ingredient and excipients with the granulation liquid are fluid bed granulation or one pot granulation.

In case of roller compaction, or in other words dry granulation, either a mixture of amlodipine or a pharmaceutically acceptable salt thereof with a part of the excipients used in the direct compression process, or the complete mixture containing all excipients, is processed through a conventional roller compactor to form ribbons, which are thereafter screened down to granules which are optionally mixed with other excipients, like glidants, lubricants and antiadherents.

First and second tablet layer compositions as described above can be compressed into bilayer tablets of the target tablet weight with appropriate size and crushing strength, using an appropriate tablet press. Optional an appropriate external lubricant spray system for the dies and punches can be used during manufacturing of tablets in order to improve lubrication.

For the production of bilayer tablets according to the present invention, the separate tablet layer compositions can be compressed in a bilayer tablet press, e.g. a rotary press in the bilayer tabletting mode, in the manner described above. In order to avoid any cross-contamination between the tablet layers (which could lead to decomposition of amlodipine), any granulate residues have to be carefully removed during tabletting by intense suction of the die table within the tableting chamber.

A method described above can be used for the manufacture of a tablet according to the present invention to treat hypertension.

In order to further illustrate the present invention, the following non-limiting examples are given:

### Formulation Examples

### Example 1: Telmisartan 80 mg / Amlodipine 10 mg 2-layer tablets

| **Constituents** | **mg per tablet** | **% of Telmisartan-layer** | **% of Amlodipine-layer** |
|---|---|---|---|
| Telmisartan | 80.000 | 16.667 | |
| Sodium hydroxide | 6.720 | 1.400 | |
| Povidone | 24.000 | 5.000 | |
| Meglumine | 24.000 | 5.000 | |
| Sorbitol | 337.280 | 70.267 | |
| Magnesium stearate | 8.000 | 1.667 | |
| Purified water * | * | * | |
| **Total Telmisartan-layer** | **480.000** | **100.000** | |
| Amlodipine mesylate | 12.800 | | 6.400 |
| Microcrystalline cellulose | 100.000 | | 50.000 |
| Dibasic calcium phosphate | 79.700 | | 39.850 |
| Sodium starch glycolate | 6.000 | | 3.000 |
| Magnesium stearate | 1.500 | | 0.750 |
| **Total Amlodipine-layer** | **200.000** | | **100.000** |
| | | | |
| **Total 2-layer tablet** | **680.000** | | |
| * Volatile component, does not remain in final product | | | |

### Example 2: Telmisartan 80 mg / Amlodipine 5 mg 2-layer tablets

| **Constituents** | **mg per tablet** | **% of Telmisartan-layer** | **% of Amlodipine-layer** |
|---|---|---|---|
| Telmisartan | 80.000 | 16.667 | |
| Sodium hydroxide | 6.720 | 1.400 | |
| Povidone | 24.000 | 5.000 | |
| Meglumine | 24.000 | 5.000 | |
| Sorbitol | 337.280 | 70.267 | |
| Magnesium stearate | 8.000 | 1.667 | |
| Purified water * | * | * | |
| **Total Telmisartan -layer** | **480.000** | **100.000** | |
| Amlodipine besylate | 6.944 | | 3.472 |
| Microcrystalline cellulose | 100.000 | | 50.000 |
| Dibasic calcium phosphate | 85.556 | | 42.778 |
| Sodium starch glycolate | 6.000 | | 3.000 |
| Magnesium stearate | 1.500 | | 0.750 |
| **Total Amlodipine-layer** | **200.000** | | **100.000** |
| | | | |
| **Total 2-layer tablet** | **680.000** | | |
| * Volatile component, does not remain in final product | | | |

### Example 3: Telmisartan 80 mg / Amlodipine 2.5 mg 2-layer tablets

| **Constituents** | **mg per tablet** | **% of Telmisartan-layer** | **% of Amlodipine-layer** |
|---|---|---|---|
| Telmisartan | 80.000 | 16.667 | |
| Sodium hydroxide | 6.720 | 1.400 | |
| Povidone | 24.000 | 5.000 | |
| Meglumine | 24.000 | 5.000 | |
| Sorbitol | 337.280 | 70.267 | |
| Magnesium stearate | 8.000 | 1.667 | |
| Purified water * | * | * | |
| **Total Telmisartan -layer** | **480.000** | **100.000** | |
| Amlodipine mesylate | 3.200 | | 1.600 |
| Microcrystalline cellulose | 100.000 | | 50.000 |
| Dibasic calcium phosphate | 89.300 | | 44.650 |
| Sodium starch glycolate | 6.000 | | 3.000 |
| Magnesium stearate | 1.500 | | 0.750 |
| **Total Amlodipine-layer** | **200.000** | | **100.000** |
| | | | |
| **Total 2-layer tablet** | **680.000** | | |
| * Volatile component, does not remain in final product | | | |

### Example 4: Telmisartan 40 mg / Amlodipine 10 mg 2-layer tablets

| **Constituents** | **mg per tablet** | **% of Telmisartan-layer** | **% of Amlodipine-layer** |
|---|---|---|---|
| Telmisartan | 40.000 | 16.667 | |
| Sodium hydroxide | 3.360 | 1.400 | |
| Povidone | 12.000 | 5.000 | |
| Meglumine | 12.000 | 5.000 | |
| Sorbitol | 168.640 | 70.267 | |
| Magnesium stearate | 4.000 | 1.667 | |
| Purified water * | * | * | |
| **Total Telmisartan -layer** | **240.000** | **100.000** | |
| Amlodipine maleate | 12.840 | | 3.210 |
| Microcrystalline cellulose | 200.000 | | 50.000 |
| Pregelatinized starch | 171.160 | | 42.790 |
| Sodium starch glycolate | 12.000 | | 3.000 |
| Colloidal silicon dioxide | 2.000 | | 0.500 |
| Magnesium stearate | 2.000 | | 0.500 |
| **Total Amlodipine-layer** | **400.000** | | **100.000** |
| | | | |
| **Total 2-layer tablet** | **640.000** | | |
| * Volatile component, does not remain in final product | | | |

### Example 5: Telmisartan 40 mg / Amlodipine 5 mg 2-layer tablets

| **Constituents** | **mg per tablet** | **% of Telmisartan-layer** | **% of Amlodipine-layer** |
|---|---|---|---|
| Telmisartan | 40.000 | 16.667 | |
| Sodium hydroxide | 3.360 | 1.400 | |
| Povidone | 12.000 | 5.000 | |
| Meglumine | 12.000 | 5.000 | |
| Sorbitol | 168.640 | 70.267 | |
| Magnesium stearate | 4.000 | 1.667 | |
| Purified water * | * | * | |
| **Total Telmisartan -layer** | **240.000** | **100.000** | |
| Amlodipine maleate | 6.420 | | 3.210 |
| Microcrystalline cellulose | 100.000 | | 50.000 |
| Pregelatinized starch | 85.580 | | 42.790 |
| Sodium starch glycolate | 6.000 | | 3.000 |
| Colloidal silicon dioxide | 1.000 | | 0.500 |
| Magnesium stearate | 1.000 | | 0.500 |
| **Total Amlodipine-layer** | **200.000** | | **100.000** |
| | | | |
| **Total 2-layer tablet** | **440.000** | | |
| * Volatile component, does not remain in final product | | | |

### Example 6: Telmisartan 40 mg / Amlodipine 2.5 mg 2-layer tablets

| **Constituents** | **mg per tablet** | **% of Telmisartan-layer** | **% of Amlodipine-layer** |
|---|---|---|---|
| Telmisartan | 40.000 | 16.667 | |
| Sodium hydroxide | 3.360 | 1.400 | |
| Povidone | 12.000 | 5.000 | |
| Meglumine | 12.000 | 5.000 | |
| Sorbitol | 168.640 | 70.267 | |
| Magnesium stearate | 4.000 | 1.667 | |
| Purified water * | * | * | |
| **Total Telmisartan -layer** | **240.000** | **100.000** | |
| Amlodipine maleate | 3.210 | | 3.210 |
| Microcrystalline cellulose | 50.000 | | 50.000 |
| Pregelatinized starch | 42.790 | | 42.790 |
| Sodium starch glycolate | 3.000 | | 3.000 |
| Colloidal silicon dioxide | 0.500 | | 0.500 |
| Magnesium stearate | 0.500 | | 0.500 |
| **Total Amlodipine-layer** | **100.000** | | **100.000** |
| | | | |
| **Total 2-layer tablet** | **340.000** | | |
| * Volatile component, does not remain in final product | | | |

### Example 7: Telmisartan 20 mg / Amlodipine 10 mg 2-layer tablets

| **Constituents** | **mg per tablet** | **% of Telmisartan-layer** | **% of Amlodipine-layer** |
|---|---|---|---|
| Telmisartan | 20.000 | 16.667 | |
| Sodium hydroxide | 1.680 | 1.400 | |
| Povidone | 6.000 | 5.000 | |
| Meglumine | 6.000 | 5.000 | |
| Sorbitol | 84.320 | 70.267 | |
| Magnesium stearate | 2.000 | 1.667 | |
| Purified water * | * | * | |
| **Total Telmisartan -layer** | **120.000** | **100.000** | |
| Amlodipine maleate | 12.840 | | 6.420 |
| Lactose monohydrate | 100.000 | | 50.000 |
| Microcrystalline cellulose | 74.160 | | 37.080 |
| Povidone | 6.000 | | 3.000 |
| Crospovidone | 5.000 | | 2.500 |
| Sodium stearyl fumarate | 2.000 | | 1.000 |
| Purified water * | * | | * |
| **Total Amlodipine-layer** | **200.000** | | **100.000** |
| | | | |
| **Total 2-layer tablet** | **320.000** | | |
| * Volatile component, does not remain in final product | | | |

### Example 8: Telmisartan 20 mg / Amlodipine 5 mg 2-layer tablets

| **Constituents** | **mg per tablet** | **% of Telmisartan-layer** | **% of Amlodipine-layer** |
|---|---|---|---|
| Telmisartan | 20.000 | 16.667 | |
| Sodium hydroxide | 1.680 | 1.400 | |
| Povidone | 6.000 | 5.000 | |
| Meglumine | 6.000 | 5.000 | |
| Sorbitol | 84.320 | 70.267 | |
| Magnesium stearate | 2.000 | 1.667 | |
| Purified water * | * | * | |
| **Total Telmisartan -layer** | **120.000** | **100.000** | |
| Amlodipine maleate | 6.420 | | 3.210 |
| Lactose monohydrate | 100.000 | | 50.000 |
| Microcrystalline cellulose | 80.580 | | 40.290 |
| Povidone | 6.000 | | 3.000 |
| Crospovidone | 5.000 | | 2.500 |
| Sodium stearyl fumarate | 2.000 | | 1.000 |
| Purified water * | * | | * |
| **Total Amlodipine-layer** | **200.000** | | **100.000** |
| | | | |
| **Total 2-layer tablet** | **320.000** | | |
| * Volatile component, does not remain in final product | | | |

### Example 9: Telmisartan 20 mg / Amlodipine 2.5 mg 2-layer tablets

| **Constituents** | **mg per tablet** | **% of Telmisartan-layer** | **% of Amlodipine-layer** |
|---|---|---|---|
| Telmisartan | 20.000 | 16.667 | |
| Sodium hydroxide | 1.680 | 1.400 | |
| Povidone | 6.000 | 5.000 | |
| Meglumine | 6.000 | 5.000 | |
| Sorbitol | 84.320 | 70.267 | |
| Magnesium stearate | 2.000 | 1.667 | |
| Purified water * | * | * | |
| **Total Telmisartan -layer** | **120.000** | **100.000** | |
| Amlodipine maleate | 3.210 | | 1.605 |
| Lactose monohydrate | 100.000 | | 50.000 |
| Microcrystalline cellulose | 83.790 | | 41.895 |
| Povidone | 6.000 | | 3.000 |
| Crospovidone | 5.000 | | 2.500 |
| Sodium stearyl fumarate | 2.000 | | 1.000 |
| Purified water * | * | | * |
| **Total Amlodipine-layer** | **200.000** | | **100.000** |
| | | | |
| **Total 2-layer tablet** | **320.000** | | |
| * Volatile component, does not remain in final product | | | |

### Example 10: Telmisartan 40 mg / Amlodipine 10 mg 2-layer tablets

| **Constituents** | **mg per tablet** | **% of Telmisartan-layer** | **% of Amlodipine-layer** |
|---|---|---|---|
| Telmisartan | 40.000 | 16.667 | |
| Sodium hydroxide | 3.360 | 1.400 | |
| Povidone | 12.000 | 5.000 | |
| Meglumine | 12.000 | 5.000 | |
| Sorbitol | 168.640 | 70.267 | |
| Magnesium stearate | 4.000 | 1.667 | |
| Purified water * | * | * | |
| **Total Telmisartan-layer** | **240.000** | **100.000** | |
| Amlodipine maleate | 12.840 | | 3.210 |
| Microcrystalline cellulose | 212.000 | | 53.000 |
| Pregelatinized starch | 169.160 | | 42.290 |
| Iron oxide yellow | 2.000 | | 0.500 |
| Colloidal silicon dioxide | 2.000 | | 0.500 |
| Magnesium stearate | 2.000 | | 0.500 |
| **Total Amlodipine-layer** | **400.000** | | **100.000** |
| | | | |
| **Total 2-layer tablet** | **640.000** | | |
| * Volatile component, does not remain in final product | | | |

### Example 11: Telmisartan 40 mg / Amlodipine 5 mg 2-layer tablets

| **Constituents** | **mg per tablet** | **% of Telmisartan-layer** | **% of Amlodipine-layer** |
|---|---|---|---|
| Telmisartan | 40.000 | 16.667 | |
| Sodium hydroxide | 3.360 | 1.400 | |
| Povidone | 12.000 | 5.000 | |
| Meglumine | 12.000 | 5.000 | |
| Sorbitol | 168.640 | 70.267 | |
| Magnesium stearate | 4.000 | 1.667 | |
| Purified water * | * | * | |
| **Total Telmisartan-layer** | **240.000** | **100.000** | |
| Amlodipine besylate | 6.944 | | 3.472 |
| Microcrystalline cellulose | 120.000 | | 60.000 |
| Pregelatinized starch | 70.056 | | 35.028 |
| Iron oxide red | 1.000 | | 0.500 |
| Colloidal silicon dioxide | 1.000 | | 0.500 |
| Magnesium stearate | 1.000 | | 0.500 |
| **Total Amlodipine-layer** | **200.000** | | **100.000** |
| | | | |
| **Total 2-layer tablet** | **440.000** | | |
| * Volatile component, does not remain in final product | | | |

### Example 12: Telmisartan 40 mg / Amlodipine 2.5 mg 2-layer tablets

| **Constituents** | **mg per tablet** | **% of Telmisartan-layer** | **% of Amlodipine-layer** |
|---|---|---|---|
| Telmisartan | 40.000 | 16.667 | |
| Sodium hydroxide | 3.360 | 1.400 | |
| Povidone | 12.000 | 5.000 | |
| Meglumine | 12.000 | 5.000 | |
| Sorbitol | 168.640 | 70.267 | |
| Magnesium stearate | 4.000 | 1.667 | |
| Purified water * | * | * | |
| **Total Telmisartan-layer** | **240.000** | **100.000** | |
| Amlodipine mesylate | 3.200 | | 1.600 |
| Microcrystalline cellulose | 120.000 | | 60.000 |
| Pregelatinized starch | 73.300 | | 36.650 |
| Iron oxide red | 0.500 | | 0.250 |
| Colloidal silicon dioxide | 1.000 | | 0.500 |
| Magnesium stearate | 2.000 | | 1.000 |
| **Total Amlodipine-layer** | **200.000** | | **100.000** |
| | | | |
| **Total 2-layer tablet** | **440.000** | | |
| * Volatile component, does not remain in final product | | | |

### Example 13: Telmisartan 40 mg / Amlodipine 5 mg 2-layer tablets

| **Constituents** | **mg per tablet** | **% of Telmisartan-layer** | **% of Amlodipine-layer** |
|---|---|---|---|
| Telmisartan | 40.000 | 23.529 | |
| Poloxamer | 8.000 | 4.706 | |
| Meglumine | 40.000 | 23.529 | |
| Mannitol | 80.500 | 47.353 | |
| Magnesium stearate | 1.500 | 0.883 | |
| Purified water * | * | * | |
| **Total Telmisartan-layer** | **170.000** | **100.000** | |
| Amlodipine maleate | 6.420 | | 3.210 |
| Microcrystalline cellulose | 100.000 | | 50.000 |
| Pregelatinized starch | 91.580 | | 45.790 |
| Colloidal silicon dioxide | 1.000 | | 0.500 |
| Magnesium stearate | 1.000 | | 0.500 |
| **Total Amlodipine-layer** | **200.000** | | **100.000** |
| | | | |
| **Total 2-layer tablet** | **370.000** | | |
| * Volatile component, does not remain in final product | | | |

## Claims

1. A pharmaceutical tablet comprising a first layer of telmisartan in a dissolving tablet matrix comprising a basic agent selected from alkali metal hydroxides, basic amino acids and meglumine, a water-soluble diluent and, optionally, other excipients and adjuvants and a second layer of amlodipine in a disintegrating or eroding tablet matrix

2. The tablet of claim 1, wherein telmisartan is in a substantially amorphous form.

3. The tablet of claim 1, wherein the dissolving tablet matrix has instant release characteristics.

4. The tablet of claim 1, wherein the water-soluble diluent is selected from monosaccharides like glucose; oligosaccharides like sucrose and lactose; and sugar alcohols like sorbitol, mannitol, and xylitol.

5. The tablet of claim 1, wherein the other excipients and adjuvants are selected from binders, carriers, fillers, lubricants, flow control agents, crystallization retarders, solubilizers, coloring agents, pH control agents, surfactants and emulsifiers.

6. The tablet of claim 1, wherein the first tablet layer composition of telmisartan is produced by spray-drying an aqueous solution comprising telmisartan and a basic agent to obtain a spray-dried granulate, mixing said spray-dried granulate with a water-soluble diluent to obtain a premix and mixing said premix with a lubricant to obtain a final blend.

7. The tablet of claim 1, wherein the disintegrating or eroding tablet matrix of the second layer comprises one or more fillers, a disintegrant, a lubricant and, optionally, a binder, a flow control agent or other excipients and adjuvants.

8. The tablet of claim 7, wherein the second tablet layer composition of amlodipine is manufactured by direct compression, wet granulation or a roller compaction process.

9. The tablet of claim 1, wherein the first layer contains 10-160 mg, preferably 20-80 mg or 40-80 mg telmisartan.

10. The tablet of claim 1, wherein the second layer contains 1-20 mg, preferably 2.5-10 mg amlodipine.

11. The tablet of claim 1 packaged in a moisture proof packaging material such as aluminium foil blister packs, or polypropylene tubes and HDPE bottles.

## Patentansprüche

1. Pharmazeutische Tablette, umfassend eine erste Schicht Telmisartan in einer sich auflösenden Tablettenmatrix, umfassend ein basisches Mittel, ausgewählt aus Alkalimetallhydroxiden, basischen Aminosäuren und Meglumin, ein wasserlösliches Verdünnungsmittel und gegebenenfalls andere Hilfsstoffe und Zusatzmittel, sowie eine zweite Schicht Amlodipin in einer sich zersetzenden bzw. zerfallenden oder erodierenden Tablettenmatrix.

2. Tablette nach Anspruch 1, wobei Telmisartan in einer im Wesentlichen amorphen Form vorliegt.

3. Tablette nach Anspruch 1, wobei die sich auflösende Tablettenmatrix sofortige Freisetzungscharakteristika aufweist.

4. Tablette nach Anspruch 1, wobei das wasserlösliche Verdünnungsmittel ausgewählt ist aus Monosacchariden, wie Glucose; Oligosacchariden, wie Saccharose und Lactose; und Zuckeralkoholen, wie Sorbitol, Mannitol und Xylitol.

5. Tablette nach Anspruch 1, wobei die anderen Hilfsmittel und Zusatzstoffe ausgewählt sind aus Bindemitteln, Trägern, Füllstoffen, Schmiermitteln, Fließhilfsmitteln, Kristallisationsverzögerern, Lösungshilfsmitteln, farbgebenden Mitteln, pH-Kontrollmitteln, oberflächenaktiven Mitteln und Emulgatoren.

6. Tablette nach Anspruch 1, wobei die erste Tablettenschichtzusammensetzung von Telmisartan hergestellt wird durch Sprühtrocknen einer wässerigen Lösung, umfassend Telmisartan und ein basisches Mittel, um ein sprühgetrocknetes Granulat zu erhalten, Mischen des sprühgetrockneten Granulats mit einem wasserlöslichen Verdünnungsmittel, um eine Vormischung zu erhalten, und Mischen der Vormischung mit einem Schmiermittel, um eine endgültige Mischung zu erhalten.

7. Tablette nach Anspruch 1, wobei die sich zersetzende bzw. zerfallende oder erodierende Tablettenmatrix der zweiten Schicht ein oder mehrere Füllstoffe, ein Zerfallshilfsmittel bzw. Sprengmittel, ein Schmiermittel und gegebenenfalls ein Bindemittel, ein Fließhilfsmittel oder andere Hilfsmittel und Zusatzstoffe aufweist.

8. Tablette nach Anspruch 7, wobei die zweite Tablettenschichtzusammensetzung von Amlodipin hergestellt wird durch direkte Kompression, Nassgranulation oder ein Walzenkompaktierverfahren.

9. Tablette nach Anspruch 1, wobei die erste Schicht 10 - 160 mg, bevorzugt 20 - 80 mg oder 40 - 80 mg Telmisartan enthält.

10. Tablette nach Anspruch 1, wobei die zweite Schicht 1 - 20 mg, bevorzugt 2,5 - 10 mg Amlodipin enthält.

11. Tablette nach Anspruch 1, verpackt in einem feuchtigkeitsdichten Verpackungsmaterial, wie Aluminiumfolienblisterverpackungen oder Polypropylenröhrchen und HDPE-Flaschen.

## Revendications

1. Comprimé pharmaceutique comprenant une première couche de telmisartan dans une matrice de dissolution pour comprimé comprenant un agent basique sélectionné parmi les hydroxydes de métaux alcalins, les acides aminés basiques et la méglumine, un diluant hydrosoluble et, éventuellement, d'autres excipients et adjuvants et une seconde couche d'amlodipine dans une matrice de délitement ou d'érosion pour comprimé.

2. Comprimé selon la revendication 1, dans lequel le telmisartan est sous une forme essentiellement amorphe.

3. Comprimé selon la revendication 1, dans lequel la matrice de dissolution pour comprimé présente des caractéristiques de libération immédiate.

4. Comprimé selon la revendication 1, dans lequel le diluant hydrosoluble est sélectionné parmi les monosaccharides comme le glucose ; les oligosaccharides comme le saccharose et le lactose ; et les alcools de sucre comme le sorbitol, le mannitol et le xylitol.

5. Comprimé selon la revendication 1, dans lequel les autres excipients et adjuvants sont sélectionnés parmi les liants, les supports, les charges, les lubrifiants, les agents de régulation d'écoulement, les retardateurs de cristallisation, les agents de solubilisation, les agents colorants, les agents de régulation du pH, les tensioactifs et les émulsifiants.

6. Comprimé selon la revendication 1, dans lequel la composition de telmisartan de la première couche du comprimé est produite par le séchage par pulvérisation d'une solution aqueuse comprenant du telmisartan et un agent basique pour obtenir des granulés séchés par pulvérisation, le mélange desdits granulés séchés par pulvérisation avec un diluant hydrosoluble pour obtenir un prémélange et le mélange dudit prémélange avec un lubrifiant pour obtenir un mélange homogène final.

7. Comprimé selon la revendication 1, dans lequel la matrice de délitement ou d'érosion pour comprimé de la seconde couche comprend une ou plusieurs charges, un agent de délitement, un lubrifiant et, éventuellement, un liant, un agent de régulation d'écoulement et d'autres excipients et adjuvants.

8. Comprimé selon la revendication 7, dans lequel la composition d'amlodipine de la seconde couche du comprimé est fabriquée par un procédé de compression directe, de granulation par voie humide ou de compactage au rouleau.

9. Comprimé selon la revendication 1, dans lequel la première couche contient 10 mg à 160 mg, de préférence 20 mg à 80 mg ou 40 mg à 80 mg de telmisartan.

10. Comprimé selon la revendication 1, dans lequel la seconde couche contient 1 mg à 20 mg, de préférence 2,5 mg à 10 mg d'amlodipine.

11. Comprimé selon la revendication 1, conditionné dans un matériau d'emballage étanche à l'humidité comme des emballages-coques à feuille d'aluminium, ou des tubes en polypropylène et des bouteilles en HDPE.
